Europäisches Patentamt

European Patent Office

Office européen des brevets

(19)

(11) **EP 1 600 772 A1**

(12) **EUROPÄISCHE PATENTANMELDUNG**

(43) Veröffentlichungstag:
**30.11.2005 Patentblatt 2005/48**

(51) Int Cl.7: **G01N 33/00**, G01N 1/22

(21) Anmeldenummer: **04405319.7**

(22) Anmeldetag: **24.05.2004**

(84) Benannte Vertragsstaaten:
**AT BE BG CH CY CZ DE DK EE ES FI FR GB GR
HU IE IT LI LU MC NL PL PT RO SE SI SK TR**
Benannte Erstreckungsstaaten:
**AL HR LT LV MK**

(71) Anmelder: **Hochschule Rapperswil,
Institut für angewandte Umwelttechnik
8640 Rapperswil (CH)**

(72) Erfinder:
• **Bunge, Rainer Prof. Dr.
8057 Zürich (CH)**
• **Ebneter, Dominik
9043 Trogen (CH)**
• **Zürcher, Markus
8640 Rapperswil (CH)**

(54) **Abgasverdünner**

(57)　Die Erfindung betrifft ein Verfahren und eine Vorrichtung zur Verdünnung von Gasen, insbesondere von heissen, wasserdampfhaltigen Abgasen mit trockener Luft. Nach dem erfindungsgemässen Verfahren wird Verdünnungsluft mit einem vorgegebenen Massenstrom Q1 über die Leitung L1 in eine Gas-Mischkammer (MK) eingeleitet und das verdünnte Messgas mit einem Massenstrom Q2 aus dieser Kammer über die Leitung L2 abgezogen. Hierbei ist Q2 grösser als Q1. Kennzeichnend ist der Umstand, dass an den Bilanzierungsstellen (B1,B2) gleicher Druck und gleiche Temperatur herrschen. Mit den an den Bilanzierungsstellen gemessenen Volumenströmen (V1,V2) lässt sich das Verdünnungsverhältnis durch folgenden Ausdruck darstellen q=1-(Q1/Q2)=1-(V1/V2). Der Verdünnungsfaktor ist also unabhängig von der Temperatur des durch die Leitung L3 in die Mischkammer eingesaugten Rohgasmassenstroms Q3. Die in Abgasen häufig unvermeidlichen Schwankungen der Temperatur des Rohgases haben keine Auswirkung auf den eingestellten Verdünnungsfaktor. Die erfindungsgemässe Vorrichtung ist robust und kompakt. Sie eignet sich insbesondere für den mobilen Feldeinsatz.

FIGUR 2

**Beschreibung**

Die Erfindung fällt in das Gebiet der Abgasmesstechnik. Sie betrifft ein Verfahren und eine Vorrichtung nach den Oberbegriffen der entsprechenden, unabhängigen Patentansprüche. Verfahren und Vorrichtung dienen zur Verdünnung von Gasen, insbesondere von heissen, wasserdampfhaltigen Abgasen mit trockener Luft.

Die Bestimmung der Konzentrationen wasserlöslicher Abgaskomponenten in einem heissen Rohabgas ist ein branchenbekanntes Problem. Viele Messgeräte haben Arbeitstemperaturen, die unterhalb des Taupunktes des Rohabgases liegen. Wird das Rohabgas zwecks Messung abgekühlt, so bildet sich ein wässriges Kondensat, in dem undefinierte Mengen der zu bestimmenden wasserlöslichen Gase absorbiert werden. Auf diese Weise ergeben sich Messwerte für die Konzentrationen dieser Gase im Abgas, die systematisch zu tief liegen.

Zur Lösung dieses Problems sind mehrere Lösungsansätze bekannt. Eine Möglichkeit ist die Verdünnung des heissen Rohabgases mit getrockneter Umgebungsluft. So kann der Taupunkt des Messgases auf eine Temperatur verringert werden, die unterhalb der Messtemperatur liegt. Zur Korrektur des Messergebnisses um den Verdünnungsfaktor ist es allerdings erforderlich diesen Verdünnungsfaktor zu bestimmen.

Viele Abgas-Verdünner arbeiten nach dem Prinzip der kritischen Venturi-Düse. Diese Technik erfordert allerdings eine relativ umfangreiche Peripherie, insbesondere eine Druckluftquelle. Solche Geräte sind insbesondere für einen mobilen Einsatz kaum geeignet.

Andere Verfahren beruhen auf dem Einsatz von Pumpen, mit denen Rohabgas und Verdünnungsluft in definierten Mengen einer Mischkammer zugeführt und als verdünntes Messgas aus dieser Mischkammer abgezogen werden.

Eine Variante dieser Verfahrensgruppe wird in EP 0 471 174 B1 ausgeführt. Im wesentlichen handelt es sich um eine Mischkammer, in die das Rohabgas und die Verdünnungsluft einströmen und aus der das verdünnte Messgas abgezogen wird. Die Massenströme des Rohabgases und der Verdünnungsluft werden bestimmt und daraus der Verdünnungsfaktor errechnet. Nachteilig ist bei diesem Verfahren, dass der Massenstrom des heissen Rohabgases exakt bestimmt werden muss - dies ist messtechnisch aufwändig.

Eine zweite Variante dieser Verfahrensgruppe ist in WO 02/070116 A1 dargestellt. Hier wird ebenfalls das verdünnte Messgas aus der Mischkammer abgezogen. Durch den entstehenden Unterdruck strömen Rohabgas und Verdünnungsluft über spezielle Düsen in die Mischkammer. Das Verdünnungsverhältnis ist durch eine Veränderung des Verhältnisses der offenen Querschnitte von Rohabgasdüsen und Verdünnungsluftdüsen einstellbar. Diese Vorrichtung ist technisch einfach, aber wenig robust. Sie ist insbesondere empfindlich gegenüber Druckschwankungen im Rohabgasstrom. Vor allem dann, wenn es sich bei dem Rohabgas um ein Dieselabgas handelt, muss zur Vermeidung einer Verstopfung der Düsen ein Russfilter im Rohabgasstrom vorgesehen werden. Dieser wiederum führt mit fortschreitender Beladung durch Russpartikel zu einem Druckabfall in der Mischkammer - die Verdünnung wird grösser und das Messergebnis wird verfälscht.

Eine dritte Variante dieser Verfahrensgruppe ist in EP 0 611 962 B1 offenbart. Die dort ausgeführte Vorrichtung ist zwar zur Messung von Partikeln in Abgasströmen vorgesehen, würde sich jedoch grundsätzlich auch zur Messung von wasserlöslichen Gasen in heissen Abgasen eignen. Hier wird die Verdünnungsluft mit einer konstanten Menge in die Mischkammer gepumpt und das verdünnte Messgas mit einer variablen Menge aus der Mischkammer abgezogen. Sowohl bei der Verdünnungsluft, als auch beim verdünnten Messgas werden Druck, Temperatur und Volumenstrom gemessen. Die Messwerte werden an einen Computer geleitet, der das aktuelle Verdünnungsverhältnis ausrechnet und die Pumpe für das verdünnte Messgas nachregelt. Dieses Verfahren erfordert aufwändige Messtechnik und Computerunterstützung, denn bei Schwankungen des Druckes oder der Temperatur im Rohabgas muss das Verdünnungsverhältnis durch Regelung der Messgaspumpe konstant gehalten werden. Auch dieses Gerät ist kaum für den tragbar-mobilen Einsatz im Feld geeignet.

Der Erfindung liegt die Aufgabe zugrunde, ein Verfahren und eine Vorrichtung zu schaffen, die es erlauben, heisse Abgase mit trockener Luft zu verdünnen. Das Verfahren und die Vorrichtung sollen technisch einfach und robust sein und überdies in einem kleinen, tragbar-mobilen Gerät untergebracht werden. Die Vorrichtung soll insbesondere auch zur Messung von $NO_2$ im russhaltigen Abgas von Dieselmotoren benutzt werden können.

Diese Aufgabe wird gelöst durch das Verfahren und die Vorrichtung, wie sie in den Patentansprüchen definiert sind.

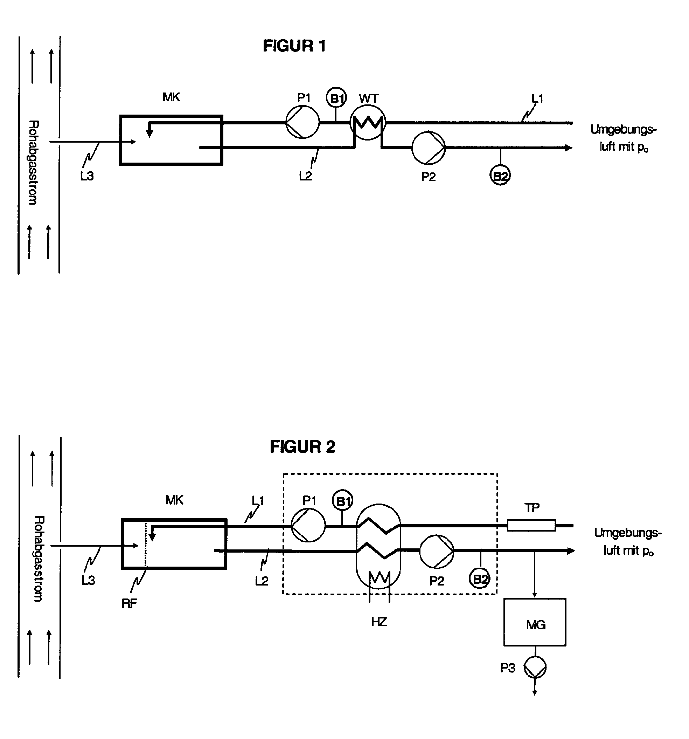

Die erfindungsgemässe Vorrichtung weist die in Figur 1 dargestellten Elemente auf. Eine Mischkammer (MK) ist mit einer Zuleitung für die Verdünnungsluft (L1), einer Ableitung für das verdünnte Messgas (L2) und einer Saugleitung für das Rohgas (L3) verbunden. Die Verdünnungsluft wird durch die Pumpe (P1) gefördert und das verdünnte Messgas über eine zweite Pumpe (P2) abgezogen. An den Bilanzierungsstellen B1 und B2 werden die Volumenströme V1 und V2 gemessen. An diesen Stellen herrscht gleicher Druck (Umgebungsdruck) und gleiche Temperatur. Temperaturgleichheit wird gewährleistet, indem die Zuleitung zu B 1 über einen Wärmetauscher (WT) mit der Zuleitung von B2 verbunden ist.

Nach dem erfindungsgemässen Verfahren wird die Verdünnungsluft mit einem vorgegebenen Massenstrom Q1 über die Leitung L1 in eine Gas-Mischkammer (MK) eingeleitet und das verdünnte Messgas mit einem Massenstrom Q2 aus dieser Kammer über die Leitung L2 abgezogen. Hierbei ist Q2 grösser als Q1. Aus der Massenbilanz ergibt sich der in die Kammer einströmende Rohgas-Teilstrom zu Q3 = Q2-Q1. Damit beträgt der Verdünnungsfaktor

$$q = Q3/Q2 = (Q2-Q1)/Q2 = 1-(Q1/Q2) \quad (Gl.1)$$

Besondere Beachtung verdient der Umstand, dass der Verdünnungsfaktor unabhängig ist von Druck und Temperatur des Rohgasmassenstroms Q3. Die in Abgasen häufig unvermeidlichen Schwankungen insbesondere der Temperatur des Rohgases haben keine Auswirkung auf den eingestellten Verdünnungsfaktor.

Kennzeichnend für unsere Erfindung ist der Umstand, dass an den Bilanzierungsstellen (B1, B2) gleicher Druck und gleiche Temperatur herrscht. Damit lässt sich das Verdünnungsverhältnis durch folgenden Ausdruck darstellen

$$q = 1-(Q1/Q2) = 1-(V1/V2) \quad (Gl.2)$$

Damit der in den Pumpen P1 und P2 geförderte Volumenstrom möglichst unabhängig vom Druck des Rohabgases bzw. vom Druck in der Mischkammer ist, werden mit Vorteil Kolbenpumpen, Membranpumpen oder Schlauchquetschpumpen verwendet.

In Figur 2 ist eine bevorzugte Ausführungsform der erfindungsgemässen Vorrichtung dargestellt. Diese Vorrichtung ist für die Verdünnung von russbeladenen Dieselabgasen geeignet. Die verwendeten Kolbenmembranpumpen haben baugleiche Pumpenköpfe die von einem gemeinsamen Motor angetrieben werden. Das Fördervolumen wird über die Hubamplitude eingestellt. In der Mischkammer befindet sich ein Russfilter (RF). In der Leitung L1 ist eine mit Silica-Gel gefüllte Trockenpatrone (TP) für die angesaugte Verdünnungsluft angebracht. Anstatt eines Wärmetauschers ist eine Heizung mit Fremdenergie vorgesehen - die Teile der Vorrichtung innerhalb des gestrichelten Rahmens sind nach aussen hin thermisch isoliert und werden auf gleicher Temperatur gehalten. So wird auch mit sehr kalter Verdünnungsluft vermieden, dass das Messgas sich soweit abkühlt, dass sich trotz der Verdünnung mit trockener Luft Kondensat in den Leitungen bildet. Das verdünnte Messgas strömt gegen Umgebungsdruck aus. Ein Teilstrom wird von der Pumpe P3 abgesaugt und durch das Messgerät (MG) geleitet. Obwohl sich der Russfilter mit fortschreitender Messdauer zusetzt und sich daher ein Unterdruck in der Mischkammer bildet, bleibt das Verdünnungsverhältnis konstant.

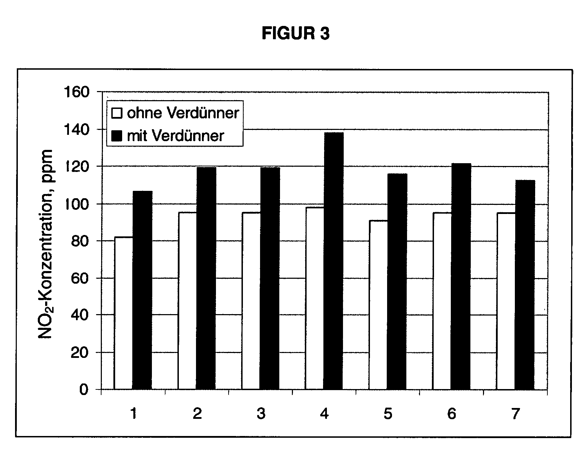

Die Vorteile des erfindungsgemässen Verfahrens seien an folgendem Bespiel erläutert. Im Abgas eines Dieselmotors sollte N02, ein wasserlösliches Gas, gemessen werden. Hierzu wurde zunächst ein konventionelles Messgerät verwendet, welches das Abgas abkühlt und die NO2-Konzentration im kalten Abgasstrom misst. Bei der Abkühlung des Rohabgases bildete sich ein Kondensat, das in dem eingebauten Kondensatabscheider gefasst wurde. Ueber den Verlauf vom mehreren Stunden wurden sieben Messungen durchgeführt und die in der Figur 3 dargestellten Werte dabei erhalten ("ohne Verdünner"). Unmittelbar nach jeder Messung wurde der erfindungsgemässe Messgasverdünner mit einem Verdünnungsverhältnis Messgas : Luft von 1: 6.25 zwischengeschaltet und das so verdünnte Messgas wiederum auf dem gleichen Messgerät gemessen. Die um den Verdünnungsfaktor 6.25 korrigierten Resultate sind ebenfalls in Figur 3 dargestellt ("mit Verdünner"). Durch Vergleich der Resultate ergibt sich, dass die ohne Messgasverdünner gemessenen Werte um etwa 25% zu niedrig sind. Die Erklärung hierfür ist, dass sich ein Teil des wasserlöslichen N02 aus dem Rohgas im Kondensat gelöst hat und so aus dem Messgasstrom entfernt wurde. Die NO2-Konzentration in dem mittels Verdünner gemessenen Abgasstrom wurde korrekt bestimmt, da durch die Verdünnung der Taupunkt des Abgases soweit herabgesetzt wurde, dass sich kein Kondensat bilden konnte.

Das erfindungsgemässe Verfahren hat überdies noch folgende Vorteile. Durch Einstellung gleicher Druck- und Temperaturbedingungen an den Bilanzierungsstellen ist der Verdünnungsfaktor unabhängig von Druck und Temperatur im Rohabgas. Im Vergleich mit EP 0 611 962 B 1 ist weder Messtechnik zur Druck- und Temperaturerfassung in L1 und L2 erforderlich, noch ein Rechner und eine davon angesteuerte Einstellung von Pumpen und Armaturen notwendig. Die Messtechnik beschränkt sich auf die Messung der Volumenströme an den Bilanzierungsstellen. Auch auf diese kann verzichtet werden, wenn die Vorrichtung mit einem Eichgas bekannter Konzentration C3 kalibriert wird. Hierbei wird die Konzentration des Eichgases im verdünnten Messgasstrom C2 gemessen. Der Verdünnungsfaktor ergibt sich aus q=C3/C2 und kann über eine der beiden Pumpen P1 oder P2 eingestellt werden. In diesem Fall hat das Verfahren den besonderen Vorteil dass diese Kalibrierung "kalt", also mit Eichgas bei Umgebungstemperatur durchgeführt werden kann.

Die erfindungsgemässe Vorrichtung ist robust und kompakt. Sie eignet sich insbesondere für den mobilen Feldeinsatz und wurde von uns in der in Figur 2 dargestellten Ausführungsform bereits als Prototyp gefertigt und erprobt.

**Patentansprüche**

**1.** Verfahren zur Verdünnung von heissen Rohabgasen mit Luft wobei

• Verdünnungsluft mit einem Massenstrom Q1 in eine Gas-Mischkammer gepumpt wird,

- das verdünnte Messgas mit einem Massenstrom Q2 aus dieser Mischkammer abgesaugt wird

- Q2 grösser ist als Q1, sodass durch den entstehenden Unterdruck ein Teilstrom des Rohabgases mit einem Massenstrom Q3 in die Mischkammer eingesaugt wird,

  *dadurch gekennzeichnet,* **dass** der Druck und die Temperatur an den Bilanzierungsstellen (B1, B2) ungefähr gleich sind.

2. Verfahren nach Anspruch 1 **dadurch gekennzeichnet, dass** an den Bilanzierungsstellen (B1, B2) die Verdünnungsluft und das verdünnte Messgas etwa unter Umgebungsdruck stehen.

3. Verfahren nach Anspruch 1 oder 2 **dadurch gekennzeichnet, dass** der Verdünnungsfaktor q = Q2/Q3 zwischen 1:1 und 30:1 liegt, vorzugsweise zwischen 2:1 und 15:1.

4. Verfahren nach einem der Ansprüche 1 bis 3 **dadurch gekennzeichnet, dass** der Verdünnungsfaktor q=V2/(V2-V1) durch Messung der Volumenströme V1 und V2 an den Bilanzierungsstellen (B1, B2) bestimmt wird.

5. Verfahren nach einem der Ansprüche 1 bis 4 **dadurch gekennzeichnet, dass** der Verdünnungsfaktor q=C3/C2 durch Verwendung von einem Rohgas-Standard bekannter Konzentration C3 und durch Messung der Konzentration C2 des Standard-Gases im verdünnten Messgas bestimmt wird.

6. Verfahren nach einem der Ansprüche 1 bis 5 **dadurch gekennzeichnet, dass** in dem verdünnten Messgas oder in einem Teilstrom davon die Konzentration eines wasserlöslichen Gases gemessen wird, welches insbesondere aus der folgenden Gruppe stammt: $NO_2$, $CO_2$, $NH_3$, $SO_2$, $H_2S$, HCN.

7. Vorrichtung zur Verdünnung von heissen Rohabgasen mit Luft welche Vorrichtung eine Gas-Mischkammer (MK) enthält, mit der drei Leitungen (L1, L2, L3) verbunden sind, wobei

- die erste Leitung L1 von einer Pumpe P1 kommt, die Luft mit einem Massenstrom Q1 in die Mischkammer fördert,

- die zweite Leitung L2 zu einer Pumpe P2 führt, die Gas mit einem Massenstrom Q2 aus der Mischkammer herausbefördert, welcher grösser ist als Q1,

- die dritte Leitung L3 vom Rohgasstrom in die Mischkammer (MK) führt

*gekennzeichnet dadurch,* **dass** die Gase in den Leitungen L1 und L2 an den Bilanzierungsstellen (B1, B2) mit der Atmosphäre im direkten Druckausgleich stehen und dass diese Vorrichtung eine Einrichtung enthält mit der die Temperatur an diesen Bilanzierungsstellen (B1, B2) auf etwa den gleichen Wert eingestellt wird.

8. Vorrichtung nach Anspruch 7 **dadurch gekennzeichnet, dass** diese Einrichtung zur Temperatureinstellung ein Wärmetauscher ist, oder eine Heizeinrichtung.

9. Vorrichtung nach einem der Ansprüche 7 oder 8 **dadurch gekennzeichnet, dass** die Pumpen P1 und P2 Kolbenpumpen, Membranpumpen, oder Schlauchquetschpumpen sind.

10. Vorrichtung nach einem der Ansprüche 7 bis 9 **dadurch gekennzeichnet, dass** an den Bilanzierungsstellen in den Leitungen L1 und L2 Messeinrichtungen für die geförderten Volumenströme angebracht sind.

11. Anwendung des Verfahrens nach den Ansprüchen 1 bis 6 zur Messung der Konzentration eines wasserlöslichen Gases, insbesondere $NO_2$, das in einem heissen wasserdampfhaltigen Abgasstrom vorliegt, vorzugsweise in einem Dieselabgas.

## FIGUR 1

Rohabgasstrom

MK   P1   B1   WT   L1

L3   L2   P2   B2

Umgebungsluft mit $p_0$

## FIGUR 2

Rohabgasstrom

MK   L1   P1   B1   TP

L3   L2   P2   B2

RF   HZ

Umgebungsluft mit $p_0$

MG

P3

**FIGUR 3**

**Europäisches Patentamt**

# EUROPÄISCHER RECHERCHENBERICHT

Nummer der Anmeldung

EP 04 40 5319

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| D,Y | EP 0 611 962 A (IVECO FIAT ; CONTROL SISTEM S R L (IT)) 24. August 1994 (1994-08-24) * Zusammenfassung; Abbildung 1 * * Spalte 2, Zeile 36 - Spalte 3, Zeile 13 * ----- | 1-11 | G01N33/00 G01N1/22 |
| Y | PATENT ABSTRACTS OF JAPAN Bd. 0113, Nr. 54 (P-638), 19. November 1987 (1987-11-19) & JP 62 133336 A (NIPPON SOKEN INC; others: 01), 16. Juni 1987 (1987-06-16) * Zusammenfassung * ----- | 1-11 | |
| A | US 5 218 857 A (DECKER HANS-JOSEF ET AL) 15. Juni 1993 (1993-06-15) * Zusammenfassung; Abbildung 1 * * Spalte 1, Zeile 41 - Zeile 48 * ----- | 1-11 | |
| A | US 6 112 575 A (COCCONI ALAN G) 5. September 2000 (2000-09-05) * Zusammenfassung; Abbildungen 1,2 * * Spalte 2, Zeile 61 - Spalte 3, Zeile 18 * ----- | 1-11 | RECHERCHIERTE SACHGEBIETE (Int.Cl.7) G01N |

Der vorliegende Recherchenbericht wurde für alle Patentansprüche erstellt

| Recherchenort | Abschlußdatum der Recherche | Prüfer |
|---|---|---|
| München | 8. Oktober 2004 | Hanisch, C |

EPO FORM 1503 03.82 (P04C03)

**ANHANG ZUM EUROPÄISCHEN RECHERCHENBERICHT**
**ÜBER DIE EUROPÄISCHE PATENTANMELDUNG NR.**

EP 04 40 5319

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

08-10-2004

| Im Recherchenbericht angeführtes Patentdokument | Datum der Veröffentlichung | Mitglied(er) der Patentfamilie | Datum der Veröffentlichung |
|---|---|---|---|
| EP 0611962 A | 24-08-1994 | IT 1260889 B<br>AT 165663 T<br>DE 69409849 D1<br>DE 69409849 T2<br>EP 0611962 A1<br>ES 2115913 T3 | 29-04-1996<br>15-05-1998<br>04-06-1998<br>03-09-1998<br>24-08-1994<br>01-07-1998 |
| JP 62133336 A | 16-06-1987 | JP 1842788 C<br>JP 5046894 B | 12-05-1994<br>15-07-1993 |
| US 5218857 A | 15-06-1993 | DE 4017472 A1<br>DE 9014528 U1<br>JP 4231868 A<br>US 5419178 A | 21-11-1991<br>03-01-1991<br>20-08-1992<br>30-05-1995 |
| US 6112575 A | 05-09-2000 | KEINE | |

EPO FORM P0461

Für nähere Einzelheiten zu diesem Anhang : siehe Amtsblatt des Europäischen Patentamts, Nr.12/82